(19) Europäisches Patentamt
European Patent Office
Office européen des brevets

(11) Veröffentlichungsnummer: **0 244 711**
**A1**

(12) # EUROPÄISCHE PATENTANMELDUNG

(21) Anmeldenummer: **87106007.5**

(22) Anmeldetag: **24.04.87**

(51) Int. Cl.⁴: **C 07 C 99/00**, C 07 C 101/18

(30) Priorität: **09.05.86 DE 3615569**

(43) Veröffentlichungstag der Anmeldung: **11.11.87**
**Patentblatt 87/46**

(84) Benannte Vertragsstaaten: **BE CH DE FR GB IT LI NL**

(71) Anmelder: **HOECHST AKTIENGESELLSCHAFT,**
**Postfach 80 03 20, D-6230 Frankfurt am Main 80 (DE)**

(72) Erfinder: **Jägers, Erhard, Dr., Hemberger Strasse 75,**
**D-5303 Bornheim (DE)**
Erfinder: **Gehrmann, Klaus, Dr.,**
**Geschwister-Scholl-Strasse 32, D-5042 Erftstadt (DE)**
Erfinder: **Koll, Hans-Peter, Ortshofstrasse 24,**
**D-5030 Hürth (DE)**

(54) **Verfahren zur Herstellung von Aminosäureestern.**

(57) Zur Herstellung von Aminosäureestern aus N-acylierten Aminosäureestern setzt man N-acylierte Aminosäureester vom Typ

```
    H
    |
R'-C-COOR'''
    |
    NH
    |
    COR''
```

wobei R', R'' und R''' beliebige, wenigstens teilweise gleiche oder verschiedene aliphatische, aromatische, araliphatische oder heteroaromatische, substituierte oder unsubstituierte Reste sind, wasserfrei mit einer Stärke und einem Alkohol um. Dabei wird ein dem Esteralkohol entsprechender Alkohol R'''OH verwendet. Als Säure kann eine Mineralsäure, ein Ionenaustauscher oder eine Lewis-Säure dienen.

EP 0 244 711 A1

Verfahren zur Herstellung von Aminosäureestern

Die vorliegende Erfindung betrifft ein Verfahren zur Herstellung von Aminosäureestern aus N-acylierten Aminosäureestern.

Bei der Synthese von Aminosäureestern fallen häufig N-acylierte Derivate als Synthesezwischenprodukte an, aus denen der Ester durch gezielte Deacylierung gewonnen werden muß.

Es ist bekannt, sowohl Ester als auch Amide mit Hilfe von wäßrigen Säuren zu den zugrundeliegenden Säuren zu hydrolysieren (vergl. J. MARCH: "Advanced Organic Chemistry", 3. Auflage, 1985, Seiten 334, 338 und 339). Wendet man dieses Hydrolyseverfahren auf N-Acylaminosäureester an, so erhält man unter gleichzeitiger Esterspaltung die freien Aminosäuren.

Es ist daher Aufgabe der vorliegenden Erfindung, ein Verfahren zur Herstellung von Aminosäureestern aus N-Acylaminosäureestern anzugeben, bei welchem die Deacylierung mit hoher Selektivität erfolgt. Das wird erfindungsgemäß dadurch erreicht, daß man N-acylierte Aminosäureester vom Typ

```
         H
         |
  R' - C - COOR'''
         |
         NH
         |
         COR''
```

wobei R', R'' und R''' beliebige, wenigstens teilweise gleiche oder verschiedene aliphatische, aromatische,

araliphatische oder heteroaromatische, substituierte oder unsubstituierte Reste sind, wasserfrei mit einer Säure und einem Alkohol umsetzt, wobei ein dem Esteralkohol entsprechender Alkohol R''' OH verwendet wird.

Das Verfahren gemäß der Erfindung kann weiterhin wahlweise auch noch dadurch ausgestaltet sein, daß

a) die Säure eine Mineralsäure ist;

b) die Säure ein Ionenaustauscher ist;

c) die Säure eine Lewis-Säure ist;

d) die Säure eine Konzentration von 0,1 n bis 10 n, vorzugsweise 1 n bis 4 n, aufweist;

e) die Umsetzung bei Temperaturen von 30 bis 200°C, vorzugsweise von 70 bis 130°C, vorgenommen wird;

f) die Umsetzung in einem Zeitraum von 0,25 bis 30 Stunden, vorzugsweise von 0,5 bis 3 Stunden, vorgenommen wird.

Beim erfindungsgemäßen Verfahren wird der N-acylierte Aminosäureester in einem Überschuß Esteralkohol, der gleichzeitig als Lösungsmittel dient, mit Säuren erwärmt, wobei die folgende Reaktion abläuft:

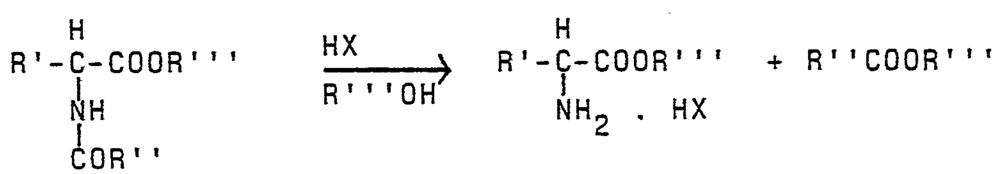

Es entsteht, ohne daß der Ester gespalten wird, das entsprechende Säuresalz des Aminosäureesters, das durch Abdampfen des Alkohols und Entfernen der Überschußsäure isoliert werden kann. Aus dem isolierten Säuresalz des Aminosäureesters kann beispielsweise durch Ammoniak der Aminosäureester freigesetzt werden.

Beim Verfahren gemäß der Erfindung sind Selektivität und Umsatz durch die Faktoren Temperatur, Verweilzeit sowie Art und Konzentration der Säure beeinflußbar, wobei der Umsatz durch Verweilzeit und Temperatur in jeder gewünschten Art steuerbar ist.

Beim erfindungsgemäßen Verfahren bieten sich als Mineralsäuren Halogenwasserstoffsäuren, Schwefelsäure und Phosphorsäure an. Die Umsetzung kann auch durch saure Ionenaustauscher oder Lewis-Säuren, beispielsweise $ZnCl_2$, $AlCl_3$ oder $BF_3$, katalysiert werden.

Beim Verfahren gemäß der Erfindung können N-acylierte Aminosäureester umgesetzt werden, welche folgende Reste aufweisen; und zwar für

R' aliphatische und heterosubstituierte aliphatische Reste (wie beispielsweise im Alanin bzw. Methionin), unsubstituierte und substituierte aromatische und araliphatische Reste (wie beispielsweise im Phenylglyzin, Phenylalanin, Tyrosin), heterocyclisch substituierte aliphatische Reste (wie beispielsweise im Tryptophan und Histidin);

R'' Acetyl- oder Benzoyl-Rest;

R''' aliphatische Reste (beispielsweise Methyl-, Ethyl-, Propyl- oder Isopropyl-), aromatische oder araliphatische Reste (beispielsweise Phenyl- oder Benzyl-).

Beim erfindungsgemäßen Verfahren können 10 bis 100 mol des dem Esteralkohol entsprechenden Alkohols R'''OH, bezogen auf den N-acylierten Aminosäureester, eingesetzt werden.

Beispiel 1 (Vergleichsbeispiel)

6 g N-Acetylphenylalaninpropylester wurden mit wäßriger 5 n Salzsäure 0,5 Stunden bei 100°C behandelt. Danach wurde das Reaktionsgemisch mit Hilfe der Gaschromatographie und der Hochdruckflüssigchromatographie untersucht. Die Analyse zeigte einen 100 %igen Umsatz zu Phenylalanin·HCl.

Beispiel 2 (gemäß der Erfindung)

6 g N-Acetylphenylalaninpropylester wurden mit propanolischer 4 n Salzsäure 2 Stunden bei 130°C behandelt. Die gemäß Beispiel 1 durchgeführte analytische Untersuchung zeigte, daß der N-Acetylalaninpropylester vollständig umgesetzt worden ist, während die Selektivität für Phenylalaninpropylester·HCl 90 % betrug.

Beispiel 3 (gemäß der Erfindung)

12 g N-Acetylphenylalaninpropylester wurden mit propanolischer 4 n Salzsäure 2 Stunden bei 100°C behandelt. Die gemäß Beispiel 1 durchgeführte analytische Untersuchung ergab einen Umsatz von 47 % bei einer Selektivität für Phenylalaninpropylester·HCl von 99 %. Der nicht umgesetzte N-Acetylphenylalaninester wurde durch Extraktion mit Toluol zurückgewonnen und in einer folgenden Charge wieder eingesetzt.

Beispiel 4 (gemäß der Erfindung)

6 g N-Acetylphenylalaninpropylester wurden mit propanolischer 4 n Salzsäure 0,25 Stunden bei 130°C behandelt. Die gemäß Beispiel 1 durchgeführte analytische Untersuchung ergab einen Umsatz von 90 % bei einer Selektivität für Phenylalaninpropylester·HCl von 90 %.

Beispiel 5 (gemäß der Erfindung)

6 g N-Acetylphenylalaninpropylester wurden mit propanolischer 1 n Salzsäure 1 Stunde bei 130°C behandelt. Die gemäß Beispiel 1 durchgeführte analytische Untersuchung ergab einen Umsatz von 50 % bei einer Selektivität für Phenylalaninpropylester·HCl von 93 %.

Beispiel 6 (gemäß der Erfindung)

6 g N-Acetylphenylalaninmethylester wurden mit methanolischer 4 n Salzsäure 2 Stunden bei 130°C behandelt. Die gemäß Beispiel 1 durchgeführte analytische Untersuchung ergab einen Umsatz von 100 % bei einer Selektivität für Phenylalaninmethylester·HCl von 88 %.

Beispiel 7 (gemäß der Erfindung)

6 g N-Acetylphenylalaninpropylester wurden mit 100 ml Propanol und 4,7 g $H_2SO_4$ 2 Stunden bei 130°C behandelt. Die gemäß Beispiel 1 durchgeführte analytische Untersuchung ergab einen Umsatz von 98 % bei einer Selektivität für Phenylalaninpropylester·Propylsulfat von 82 %.

Beispiel 8 (gemäß der Erfindung)

6 g N-Acetyltyrosinmethylester wurden mit methanolischer 4 n Salzsäure 2 Stunden bei 130°C behandelt. Die gemäß

Beispiel 1 durchgeführte analytische Untersuchung ergab einen Umsatz von 96 % bei einer Selektivität für Tyrosinmethylester·HCl von 89 %.

Beispiel 9 (gemäß der Erfindung)

2 g N-Acetylphenylalaninpropylester wurden in 50 ml Propanol mit 10 g Ionenaustauscher (® Amberlyst 15) 8 Stunden unter Rückfluß umgesetzt. Nach dem Eindampfen wurde der Ionenaustauscher mit wäßriger Salzsäure gewaschen und die resultierende Lösung gemäß Beispiel 1 analytisch untersucht. Es ergab sich ein Umsatz von 20 % bei einer Selektivität für Phenylalaninpropylester·HCl von 98 %.

HOECHST AKTIENGESELLSCHAFT HOE 86/H 012

# Verfahren zur Herstellung von Aminosäureestern

Patentansprüche:

1. Verfahren zur Herstellung von Aminosäureestern aus N-acylierten Aminosäureestern, <u>dadurch gekennzeichnet</u>, daß man N-acylierte Aminosäureester vom Typ

```
          H
          |
R' - C - COOR'''
          |
          NH
          |
          COR''
```

wobei R', R'' und R''' beliebige, wenigstens teilweise gleiche oder verschiedene aliphatische, aromatische, araliphatische oder heteroaromatische, substituierte oder unsubstituierte Reste sind, wasserfrei mit einer Säure und einem Alkohol umsetzt, wobei ein dem Esteralkohol entsprechender Alkohol R''' OH verwendet wird.

2. Verfahren nach Anspruch 1, <u>dadurch gekennzeichnet</u>, daß die Säure eine Mineralsäure ist.

3. Verfahren nach Anspruch 1, <u>dadurch gekennzeichnet</u>, daß die Säure ein Ionenaustauscher ist.

4. Verfahren nach Anspruch 1, <u>dadurch gekennzeichnet</u>, daß die Säure eine Lewis-Säure ist.

5. Verfahren nach mindestens einem der Ansprüche 1 bis 4, <u>dadurch gekennzeichnet</u>, daß die Säure eine Konzentration von 0,1 n bis 10 n, vorzugsweise 1 n bis 4 n, aufweist.

6. Verfahren nach mindestens einem der Ansprüche 1 bis 5, dadurch gekennzeichnet, daß die Umsetzung bei Temperaturen von 30 bis 200°C, vorzugsweise von 70 bis 130°C, vorgenommen wird.

7. Verfahren nach mindestens einem der Ansprüche 1 bis 6, dadurch gekennzeichnet, daß die Umsetzung in einem Zeitraum von 0,25 bis 30 Stunden, vorzugsweise von 0,5 bis 3 Stunden, vorgenommen wird.

0244711

Nummer der Anmeldung

Europäisches Patentamt

# EUROPÄISCHER RECHERCHENBERICHT

EP 87106007.5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | HOUBEN-WEYL "Methoden der organischen Chemie", 4. Auflage, Band XI/1 "Stickstoffverbindungen II, Amine" 1957<br>GEORG THIEME VERLAG, Stuttgart<br>Seite 935, Zeilen 4-24<br>-- | 1,2, 5-7 | C 07 C 99/00<br>C 07 C 101/18 |
| A | DE - C - 955 509 (FARBENWERKE HOECHST AG)<br>* Seite 2, Zeilen 29-40,92-99 *<br>-- | 1,2 | |
| A | EP - A2 - 0 077 099 (ANIC S.P.A.)<br>* Beispiel 5 *<br>---- | 1,2, 5-7 | |
| | | | RECHERCHIERTE SACHGEBIETE (Int. Cl.4) |
| | | | C 07 C 99/00<br>C 07 C 101/00 |

Der vorliegende Recherchenbericht wurde für alle Patentansprüche erstellt.

| Recherchenort | Abschlußdatum der Recherche | Prüfer |
|---|---|---|
| WIEN | 09-07-1987 | HOFBAUER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82